# EUROPEAN PATENT APPLICATION

(11) **EP 2 374 873 A1**
(43) Date of publication of application: **12.10.2011**
(21) Application number: 10003770.4
(22) Date of filing: 08.04.2010
(51) Int. Cl.: C12N 9/12, C12P 13/08

(54) **Modified aspartate kinase from corynebacterium and its application for amino acid production**

(71) Applicant: Technische Universität Hamburg-Harburg, 21073 Hamburg (DE); TuTech Innovation GmbH, 21079 Hamburg (DE)
(72) Inventor: Zeng, An-Ping, 21071 Hamburg (DE); Chen, Zhen, 21071 Hamburg (DE)
(74) Representative: UEXKÜLL & STOLBERG

(57) **Abstract**

The present invention relates to methods and enzymes which are useful for producing L-lysine and L-threonine. In particular, the present invention provides a modified aspartokinase enzyme with a reduced sensitivity to feedback inhibition by L-lysine or mixtures of L-lysine and L-threonine. The invention also provides methods for producing L-lysine and/or L-threonine which make use of the modified aspartokinase enzyme. The invention also relates to the use of the modified aspartokinase enzyme for L-lysine and/or L-threonine.

## Description

The present invention relates to methods and enzymes which are useful for producing L-lysine and L-threonine. In particular, the present invention provides a modified aspartokinase enzyme with a reduced sensitivity to L-lysine or L-lysine/L-threonine feedback inhibition which is derived from *Corynebacterium.* The invention also provides methods for producing L-lysine and/or L-threonine which make use of the modified aspartokinase enzyme. The invention also relates to the use of the modified aspartokinase enzyme for producing L-lysine and/or L-threonine.

### BACKGROUND OF THE INVENTION

Amino acids are the basic building blocks for proteins. These nutritional key compounds are widely used for the preparation of feed additives, food ingredients, nutraceuticals and pharmaceuticals. The world production of amino acids has been steadily increasing in recent years and has reached approximately 2 million tons per year. In particular, the amino acids of the aspartic acid group (including L-lysine and L-threonine) have found wide applications, and several hundred thousand tons of these amino acids are produced per year worldwide by microbial fermentation using organisms such as *Corynebacterium glutamicum* and *Escherichia coli.*

The biosynthesis of amino acids of the aspartic acid group in *E. coli* and *Corynebacterium glutamicum* follows a cascade of biochemical reactions involving the conversion of aspartic acid to β-phosphoaspartic acid. This reaction, which is regarded as a key regulatory step in the biosynthetic pathway, is catalyzed by the enzyme aspartokinase. In its wild-type form, this enzyme is subject to feedback inhibition by L-lysine or a mixture of L-lysine and L-threonine, the latter of which synergistically inhibits the activity of the enzyme. Therefore, *Corynebacterium* strains which are useful for the industrial production of L-lysine or L-threonine usually contain mutations in the lysC gene in order to provide a desensitized enzyme (see, for example, US 5,688,671). The release of aspartokinase from feedback control mechanisms is regarded as one of the most important features of industrial lysine and or threonine producer strains.

Despite several improvements, novel means and methods would be desirable which provide for a release of the biosynthesis process of producing L-lysine and/or L-threonine from feedback inhibition by L-lysine or L-lysine/L-threonine. The present invention provides novel aspartokinase enzymes which have been obtained by modifying the amino acid sequence of the aspartokinase of and *Corynebacterium glutamicum* by amino acid substitutions which differ from those described in the art. Thus, the present invention provides further alternatives for optimizing the production process of L-lysine and L-threonine. By introducing genes coding for these modified enzymes into L-lysine and/or L-threonine producing bacteria, the fermentative production of these amino acids can be significantly improved.

### DETAILED DESCRIPTION OF THE INVENTION

The present invention provides improved aspartokinase enzymes which have been derived from the *Corynebacterium glutamicum* enzyme depicted in SEQ ID NO:1 by one or more amino acid substitutions at pre-determined amino acid positions. Specifically, it was found that an amino acid substitution in any of amino acid positions 274, 298, 299, 374, 382 and 384, of the aspartokinase enzyme from *C. glutamicum* results in a modified enzyme which has a significantly reduced feedback inhibition sensitivity towards L-lysine or a combination of L-lysine and L-threonine. This means that compared to the wild-type enzyme, higher concentrations of L-lysine or mixtures of L-lysine and L-threonine are required to inhibit the enzymatical activity of the enzyme. Some of the mutated enzymes were even found to be almost completely released from feedback inhibition. Releasing the aspartokinase enzyme from feedback inhibition is a particularly suitable way of increasing the yield of the amino acids L-lysine and L-threonine in a microorganism-based production process, as aspartokinase catalyses a decisive step in the biosynthesis of both amino acids.

The amino acid sequence shown herein as SEQ ID NO:1 is the amino acid sequence of wild-type aspartokinase of *C. glutamicum.* This enzyme has been widely used in the art for the production of the amino acid L-lysine and L-threonine. In order to enhance the product yield during the production of L-lysine and L-threonine, several amino acid substitutions in the sequence of SEQ ID NO:1 have been suggested in the art for releasing the enzyme from the feedback inhibition by L-lysine or mixtures of L-lysine and L-threonine. For example, US patent 5,688,671 discloses an amino acid substitution at positions 279 (Ala to Thr) of SEQ ID NO:1. The present invention has identified additional positions which can be mutated to achieve a significant or complete release from feedback inhibition effected by L-lysine or mixtures of L-lysine and L-threonine. Therefore, the present invention provides novel enzymatically active polypeptides and enzymatically active fragments thereof which are useful for providing an enhanced production of L-lysine and L-threonine. Based on the present disclosure, genetically modified microorganisms, in particular strains of *E. coli* or *C. glutamicum,* can be generated for use in optimized fermentative production processes.

Host cells which express the aspartokinase enzyme modified according to the invention will produce both L-lysine and L-threonine upon culturing of the cells in a suitable production medium. While L-lysine is normally obtained in high yields, the amount of L-threonine recovered from the culture will be lower. Consequently, additional modifications may be required to increase the yield for L-threonine. For example, it may be required to provide the producing cells with one or more additional copies of the phosphoenolpyruvate carboxylase gene and the threonine operon, as described in US 7,011,961. In addition, it may also be required to provide for a mutated aspartokinase I enzyme which has a reduced L-threonine feedback inhibition sensitivity, as described in US 5,175,107, US 7,186,531, US 7,220,571.

In a first aspect, the invention thus provides a polypeptide which is an enzymatically active aspartokinase with a reduced sensitivity to feedback inhibition by L-lysine or mixtures of L-lysine and L-threonine, said polypeptide comprising the amino acid sequence of SEQ ID NO:1 or an enzymatically active fragment or homolog thereof, wherein one or more amino acids have been replaced at positions that correspond to amino acids 274, 298, 299, 374, 382 and 384 of SEQ ID NO:1. In general, an amino acid occurring at one of the positions which correspond to positions 274, 298, 299, 374, 382 and 384 of SEQ ID NO:1 can be replaced by any other naturally or non-naturally occurring amino acids or by an amino acid analogue, provided that the modified aspartokinase resulting from the replacement is still enzymatically active and exhibits a reduced sensitivity with respect to the feedback inhibition imposed by L-lysine or mixtures of L-lysine and L-threonine.

As the modified aspartokinase enzymes provided by the present invention are for use in production processes of L-lysine and L-threonine, it is crucial that the above-mentioned amino acid substitutions, either at positions relevant for feedback inhibition or not, do not result in a significant loss of activity of the enzyme due to a spatial rearrangement of the polypeptide structure. The polypeptides provided by the invention are enzymatically active, which means that they have retained at least part of the enzymatic activity of the aspartokinase depicted in SEQ ID NO:1 (as measured in the absence of L-lysine or mixtures of L-lysine and L-threonine). Preferably, the polypeptides provided by the present invention have retained 50%, 60%, 70%, 80%, 90%, 95%, 96%, 97%, 98%, or 99% of the activity of the aspartokinase of SEQ ID NO:1, as measured in the absence of L-lysine or mixtures of L-lysine and L-threonine. According to a particularly preferred embodiment, the amino acid replacements disclosed by the present invention, which result in a complete or partial release from feedback inhibition, have no influence on the enzymatic activity of the mutated aspartokinase.

It has been found that the aspartokinase enzymes of the invention, which have been modified by the replacement of any of the amino acids at positions 274, 298, 299, 374, 382 and 384 of SEQ ID NO:1, show a significant reduction in sensitivity to feedback inhibition by L-lysine or mixtures of L-lysine and L-threonine. Preferably, the polypeptide enzymes of the invention are at least 2-fold, at least 5-fold, at least 10-fold, at least 15-fold, at least 20-fold, at least 25-fold, at least 50-fold, at least 75-fold, at least 100-fold, at least 150-fold, at least 200-fold, at least 250-fold, at least 500-fold, at least 750-fold, at least 1000-fold, or at least 5000-fold less sensitive to feedback inhibition by L-lysine or mixtures of L-lysine and L-threonine as compared to the original enzyme shown in SEQ ID NO:1 when measured, for example, in the assay described in the examples below. Even more preferably, the polypeptides of the invention are substantially free from feedback inhibition by L-lysine or mixtures of L-lysine and L-threonine (i.e. resistant), which means that the presence of lysine in the culture medium does not impair or otherwise negatively influence the enzyme activity of the mutated aspartokinase enzymes.

The polypeptide of the invention may comprise an amino acid sequence as shown in SEQ ID NO:1, in which one or more of the amino acids located in positions 274, 298, 299, 374, 382 and 384 of SEQ ID NO:1 have been replaced by other amino acids. The amino acids which can be used for replacing the respective amino acids in the naturally occurring enzyme of *C. glutamicum* are not limited to specific amino acids. In principle, any other proteinogenic or non-proteinogenic amino acid may be used for substituting the naturally occurring amino acid in the respective position of the enzyme. Preferably, the amino acids found in the original enzyme of *C. glutamicum* can be replaced by any other naturally occurring, proteinogenic amino acid. As used herein, proteinogenic amino acids are those 23 amino acids which are regularly found in naturally occurring polypeptides, i.e. isoleucine (Ile), alanine (Ala), leucine (Leu), asparagine (Asn), lysine (Lys), aspartic acid (Asp), methionine (Met), cysteine (Cys), phenylalanine (Phe), glutamic acid (Glu), threonine (Thr), glutamine (Gln), tryptophan (Trp), glycine (Gly), valine (Val), proline (Pro), serine (Ser), tyrosine (Tyr), arginine (Arg), histidine (His), selenocysteine, selenomethionine, and pyrrollysine. Preferably, the amino acids are L-amino acids. However, also D-amino acids may be useful for replacing the amino acids in the original polypeptide of SEQ ID NO:1.

Alternatively, the amino acids used for replacing the amino acids in the naturally occurring enzyme of *C. glutamicum* are non-proteinogenic amino acids, i.e. amino acids which are not found in naturally occurring polypeptides. These non-proteinogenic amino acids include, for example, α-aminoadipic acid, β-aminoadipic acid, α-aminobutyric acid, α-aminoisobutyric acid, β-alanine, 4-aminobutyric acid, 5-aminovaleric acid, 6-aminohexanoic acid, 8-aminooctanoic acid, 9-aminononanoic acid, 10-aminodecanoic acid, 12-aminododecanoic acid, α-aminosuberic acid, β-cyclohexylalanine, citrulline, dehydroalanine, α-cyclohexylglycine, propargylglycine, pyroglutamic acid, 4-benzoylphenylalanine, δ-hydroxylysine, 4-hydroxyproline, alloisoleucine, lanthionine (Lan), norleucine, norvaline, ornithine, phenylglycin, pipecolic acid, sarcosine, 1,2,3,4-tetrahydro-isochinoline-3-carboxylic acid, allo-threonine, thiazolidine-4-carboxylic acid, y-aminobutyric acid (GABA), iso-cysteine, diaminopropionic acid, 2,4-diaminobutyric acid, 3,4-diaminobutyric acid, biphenylalanine and 4-fluoro-phenylalanine.

Also included by the term "non-proteinogenic amino acids" are derivatives of the above-mentioned proteinogenic amino acids wherein a side-chain has been modified, for example, by a methylene group, thereby providing e.g. homomethionine, homoserine, homoproline, homothreonine, homotryptophane, homotyrosine, homohistidine and homolysine. However, it is preferred according to the invention that an amino acid located in SEQ ID NO:1 at one of the positions 274, 298, 299, 374, 382 and 384 is replaced by one of the other 22 proteinogenic amino acids.

In a particularly preferred aspect of the present invention, the aspartokinase depicted in SEQ ID NO:1 has been modified by one or more of the following replacements:
(a) Asp at position 274 is modified to Ala, Ile, Leu, Gly, Phe, Trp, Tyr, Val, Pro, Cys, Met, Thr, Glu, Lys, Arg, or His;
(b) Gln at position 298 is modified to Gly, Ala, Leu, Phe, Ile, Trp, Tyr, Val, Arg, Pro, or Cys;
(c) Asn at position 299 is modified to Leu, Ala, Ile, Gly, Trp, Tyr, Ser, His, Val, Pro, or Cys;
(d) Asn at position 374 is modified to Ala, Ile, Leu, Gly, Trp, Tyr, Val, Pro, Cys, Ser, Thr, Met, Gln, Asp, Glu, Lys, Arg, Phe, His;
(e) Glu at position 382 is modified to Ala, Ile, Phe, Gly, Trp, Tyr, Pro, Ser, Leu or Cys;
(f) Arg at position 384 is modified to Leu, Ala, Ile, Gly, Phe, Trp, Tyr, Val, Pro or Cys.

In a particularly preferred embodiment, the modified aspartokinase of the present invention comprises one or more of the following amino acid replacements: (a) Asp at position 274 to Ala; (b) Gln at position 298 to Gly; (c) Asn at position 299 to Leu; (d) Asn at position 374 to Ala; (e) Glu at position 382 to Ala; (f) Arg at position 384 to Leu.

It will be appreciated that the invention is not restricted to the specific aspartokinase sequence depicted in SEQ ID NO:1. Instead, also enzymatically active homologs of the amino acid sequence of SEQ ID NO:1 may be used, which include (apart from the one or more amino acid replacements in positions 274, 298, 299, 374, 382 and 384) additional sequence differences when compared to the amino acid sequence depicted in SEQ ID NO:1. For example, the invention is suitable for use with aspartokinases from different species or strains of *Corynebacterium,* which might differ from SEQ ID NO:1 by one or more amino acid substitutions, or with enzymes isolated from a closely related genus.

Homologs of the sequence of SEQ ID NO:1 typically differ from the sequence of SEQ ID NO:1 by one or more deletions, substitutions or additions of amino acids in positions other than those corresponding to positions 274, 298, 299, 374, 382 and 384 of SEQ ID NO:1. Accordingly, apart from the residues which are relevant for the feedback inhibition, one or more further amino acids of the enzyme in SEQ ID NO:1 may be substituted or deleted as long as such modification does not impair the intended release from feedback inhibition, and the homolog resulting from the deletion or substitution is still an enzymatically active aspartokinase, i.e. the modification should not substantially diminish the enzyme activity of the aspartokinase. Generally, any amino acid residue of the amino acid sequences shown in SEQ ID NO:1 can be replaced by a different amino acid, provided that the resultant sequence of the variant is still an enzymatically active polypeptide with aspartokinase function. In particular, the enzyme depicted in SEQ ID NO:1 may be modified by the substitution of a total of 1, 2, 3, 4, 5, 6, 7, 8, 9, 10, 15, 20, 25, 30, 35, 40, 45 or 50 amino acids, and in some embodiments even in up 50, 60, 70, 80, 90 or 100 amino acids of the enzyme depicted in SEQ ID NO:1. Preferably, these substitutions are not relevant for the release from feedback inhibition by L-lysine or mixtures of L-lysine and L-threonine.

If a polypeptide is used in the practice of the invention which includes substitutions at positions other than those disclosed herein in the context with release from feedback inhibition, i.e. in positions other than those corresponding to 274, 298, 299, 374, 382 or 384 of SEQ ID NO:1, it is preferred that such substitutions are conservative substitutions, i.e. substitutions of one or more amino acid residues by an amino acid of a similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids which may be used for a conservative substitution comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine and lysine. Examples of charged polar (acidic) amino acids comprise aspartic acid and glutamic acid.

Likewise, polypeptides which differ from the sequence depicted in SEQ ID NO:1 by the insertion of one or more additional amino acids are considered homologs in the context of the present invention. Such insertions can be made at any position of the polypeptide shown in SEQ ID NO:1. Likewise, homologs also include polypeptides in which one or more amino acids have been deleted relative to the polypeptide shown in SEQ ID NO:1. In principle, such deletions can be applied to any amino acid position of the sequence of SEQ ID NO:1.

A homolog of the sequence of SEQ ID NO:1 shows a high degree of sequence identity with the sequence of SEQ ID NO:1. The amino acid identity will be at least about 75%, 80%, 85%, 90%, 91%, 92%, 93%, 94%, 95%, 96%, 97%, 98% or 99% when compared in an optimal alignment, for example, by the program BESTFIT using standard parameters. For example, a sequence identity of 90% means that 90% of the amino acids of an analyzed amino acid sequence stretch are identical to the sequence of the reference amino acid sequence depicted in SEQ ID NO:1. Methods and computer programs for determining amino acid sequence identity are well known in the art.

Also encompassed by the invention are enzymatically active fragments of the aspartokinase shown in SEQ ID NO:1 as well as enzymatically active fragments of the above-mentioned homologs of the aspartokinase shown in SEQ ID NO:1, provided that these fragments cover a sequence which includes one or more of the substitutions in positions corresponding to positions 274, 298, 299, 374, 382 and 384 in SEQ ID NO:1. Enzymatically active fragments of the sequence shown in SEQ ID NO:1 or its homologs are polypeptides that differ from the amino acid sequence shown in SEQ ID NO:1 (or from the respective homolog sequence) by the absence of one or more amino acids at the N-terminus and/or the C-terminus of the polypeptide. For example, a fragment of the sequence of SEQ ID NO:1 may differ from the sequence of SEQ ID NO:1 by the lack of about 5, 10, 15, 20, 25, 30, 35, 40, 45 or 50 amino acids at the N-terminus and/or the C-terminus, provided that such fragment retains at least a part of the enzymatic activity of the original full-length enzyme depicted in SEQ ID NO:1. Likewise, a fragment of a homolog of SEQ ID NO:1 may differ from said homolog sequence by the lack of about 5, 10, 15, 20, 25, 30, 35, 40, 45 or 50 amino acids at the N-terminus and/or the C-terminus, provided that the fragment is still enzymatically active.

The present invention also encompasses a polynucleotide encoding a polypeptide, wherein said polypeptide is an enzymatically active aspartokinase with a reduced sensitivity to L-lysine feedback inhibition, said polypeptide comprising the amino acid sequence of SEQ ID NO:1 or an enzymatically active fragment or homolog thereof. For example, the polynucleotide of the invention can comprise the complete of part of the coding region of the nucleotide sequence referred to as SEQ ID NO:7 in US Patent 5,688,671, wherein the triplets encoding amino acid positions 274, 298, 299, 374, 382 and/or 384 have been appropriately modified to provide the amino acid replacements discussed herein. Of course, the invention also encompasses polynucleotides which differ from the above-discussed polynucleotides due to the degeneracy of the genetic code.

The invention further refers to a vector, which comprises a polynucleotide coding for an aspartokinase enzyme of the invention with a reduced sensitivity to feedback inhibition by L-lysine or mixtures of L-lysine and L-threonine (or an enzymatically active fragment or homolog thereof). In one aspect of the invention, the vector is an expression vector. Generally, an expression vector refers to a polynucleotide construct, normally comprised of DNA, which allows for the genetic transfer, replication and expression of an inserted polynucleotide in a host cell, wherein said polynucleotide is not native to said host cell, or which is native to said host cell, but has been modified. Replication of the vector can be achieved either by integration of the vector into the genome of the host cell, or by episomal replication (e.g. plasmids). In the latter case, the expression vector comprises a functional origin of replication which allows for its replication in the respective host cell.

The expression vector for use in the present methods is preferably a vector having a high copy number within the host cells so as to provide for high expression levels of the aspartokinase in the host cell. The expression vector will normally comprise a gene coding for a selectable marker which is useful for identifying and selecting host cells which have been transformed with the vector. Useful markers include those which confer resistance to antibiotics, such as ampicillin, kanamycin, and tetracycline. Such markers are known in the art, and the choice of the proper marker will depend on the host cell and the vector used.

Preferred expression vectors for use in the present invention comprise a polynucleotide of the invention in a form that allows for the expression of said polynucleotide in a host cell. Normally, the expression vector will include regulatory sequences, such as promoters and enhancers, which are operatively linked to the polynucleotide selected for expression. The promoter used in the expression vector may be a constitutive or an inducible promoter. It will be appreciated that the design of the choice of the specific elements for inclusion into the expression vector will depend on the host cell to be transformed, the desired level of polypeptide expression, and the like. Preferably, the expression vectors are designed for the expression of an aspartokinase enzyme of the invention in prokaryotic cells. More preferably, the expression vectors allow for the expression of the aspartokinase of the present invention in bacterial cells, such as in cells of *E*. *coli* and *C*. *glutamicum.*

The expression vectors may also provide for the expression of the aspartokinase in the form of a fusion polypeptide. A fusion polypeptide refers to a non-naturally occurring hybrid polypeptide comprising at least two different polypeptides or polypeptide fragments which do occur naturally associated to each other. Expression vectors which result in the expression of fusion polypeptides normally add several amino acids to the polypeptide to be expressed, either at the N-terminus or at the C-terminus. The additional amino acid may be, for example, helpful for enhancing the expression of the aspartokinase polypeptide in the host cell, or for purifying said polypeptide after expression.

Also provided are host cells which contain a polynucleotide or an expression vector of the present invention. Different host cells can be used for the production of the aspartokinase of the invention from the expression vector. In general, both eukaryotic (e.g. yeasts or animal cells) and prokaryotic host cells can be used for recombinantly producing the aspartokinase. However, it is preferred that the host cell is a prokaryotic cell. Bacterial cells are particularly preferred. For example, the host cells may be bacteria of the genera Es*cherichia,* Serratia, *Brevibacterium* and *Corynebacterium,* as these bacterial hosts are frequently used in established production processes for L-lysine and L-threonine. As shown in the examples, the gene of *C. glutamicum* can readily be introduced and expressed in *E. coli.* Host cells of the genus *Escherichia* or *Corynebacterium* are the most preferred host cells according to the present invention. Strains of *E. coli* that can be used for the production of L-lysine or L-threonine comprise K-12, JM109, GT3 and the like. Strains of *Corynebacterium* include, for example, strains of *C*. *acetoacidophilum* (ATCC 13870 or ATCC 15806), *C*. *callunae* (ATCC 15991), *C. glutamicum* (ATCC 13032 or ATCC 13060), *C*. *lilium.* (ATTC 15990), *C*. *melassecola* (ATTC 17965), and the like.

The host cell which is used for the amino acid production process by fermentation may be a cell which naturally possesses all biosynthetic enzymes which are required for producing L-lysine and/or L-threonine. These cells will be able to synthesize both amino acids. For example, a wild-type *C. glutamicum* cell comprising the gene depicted in SEQ ID NO:1 may be used. However, when using the wild-type strain, the yield of L-lysine and L-threonine will be undesirably low, due to the above-discussed feedback inhibition of the aspartokinase enzyme. By transforming the cells with a polynucleotide coding for a modified aspartokinase enzyme of the invention, the yield can be significantly increased, since the modified aspartokinase enzyme has a reduced sensitivity to feedback inhibition by L-lysine or mixtures of L-lysine and L-threonine. Alternatively, the host cell used for the production process may be a cell which does not contain a *lysC* gene or which contains a gene coding for a non-functional aspartokinase. In these cells, a successful transformation with a polynucleotide of the invention would be reflected by the fact that the cells start producing the amino acids of interest.

Depending on the host organism selected for transformation, a suitable expression vector will be provided. Examples for suitable vectors for polypeptide expression in *E. coli* cells comprise, for example, the vectors of the pBluescript series, the vectors of the pUC series, e.g. pUC18, pUC19, pBR322, pBR329, pQE70, pQE60, pQE-9, pNH8A, pNH16a, pNH18A, pNH46A, ptrc99a, pKK223-3, pKK233-3, pDR540, pRIT5, pLG338, pKC30, pHSG299, pHSG399, pRep4, pACYC177, pACYC184, pRSF1010, pBW22, and the like. Examples for expression vectors suitable for *C. glutamicum* comprise, for example, pAM330, pHM1519, pAJ655, pAJ611, pAJ1844, pCG11, pCG2, pCG4 and the mobilizable *E. coli*/*C. glutamicum* shuttle vectors, e.g. pEC-XT99A, pEC-XC99E, pET-XK99E. Further examples are the vectors described, e.g. in Kirchner et al., 2003, J. Biotechnol., 104:287-299, and in "Cloning Vectors" (Pouwels et al. (eds.) Elsevier, Amsterdam New York Oxford, 1985). The expression vector may be transformed into the host cell by any suitable method. The expression vector of the invention may be introduced into the host cell, e.g. by electroporation, microinjection, particle bombardement or by chemical methods such as calcium phosphate-mediated transformation, as described in Maniatis et al. 1982, Molecular Cloning, A laboratory Manual, Cold Spring Harbor Laboratory.

In a further aspect, a polynucleotide encoding an aspartokinase of the present invention is introduced into a suitable *C. glutamicum* strain, e.g. the wild-type *C. glutamicum* ATCC 1302, by a gene replacement method. For example, the polynucleotide encoding an aspartokinase of the present invention may be introduced into the genome of the *C. glutamicum* strain by homologous recombination using vectors which do not replicate in *C. glutamicum,* such as pK18mobsacB or pK19mobsacB. By two subsequent crossover events, it is possible to replace part of the lysC wild-type gene with a corresponding part of a polynucleotide encoding an aspartokinase enzyme modified according to the invention.

In a further aspect, the invention relates to a method of producing L-lysine and/or L-threonine, comprising the step of (i) culturing a host cell as defined above in a culture medium under conditions which allow for the expression of a polypeptide which is an enzymatically active aspartokinase with a reduced sensitivity to feedback inhibition by L-lysine or mixtures of L-lysine and L-threonine, said polypeptide comprising the amino acid sequence of SEQ ID NO:1 or an enzymatically active fragment or homolog thereof, wherein one or more amino acids have been replaced at positions that correspond to amino acids 274, 298, 299, 374, 382 and 384 of SEQ ID NO:1, and (ii) obtaining L-lysine and/or L-threonine from the culture medium.

Appropriate cell culture media are well known in the art for different kinds of host cells, in particular for bacteria such as *E. coli* and *C. glutamicum.* For example, strains of *E. coli* can be conveniently grown in MB media, LB media and BHI media. A suitable standard minimal media for *E. coli* is M9. Likewise, strains of *C. glutamicum* can be grown in LB media or brain heart infusion medium. The specific media for use in culturing a host cell transformed with a polynucleotide encoding the modified aspartokinase of the invention will depend on the particular strain which is selected for the production process. The skilled person will have no problems to find appropriate media for a host cell based on the extensive literature available in the art.

Culturing of the cells expressing the aspartokinase of the invention can be performed in accordance with standard fermentation techniques. For example, the cells can be cultured in a batch or fed-batch process. Culturing methods are generally described in Encyclopedia of Bioprocess Technology - Fermentation, Biocatalysis, and Bioseparation, Volumes 1-5, Flickinger, M. C., Drew, S. W. (eds.), 1999 John Wiley & Sons. Typical temperature conditions will be in the range of 20°C to 42°C, more commonly 30°C to 40°C, and preferably 35°C to 38°C. The culture broth will typically have a pH of between 6.5 and 9.0, more typically 7.0 to 8.0, e.g. 7.5. Fermentation of the culture will be continued for a time period ranging from several hours to several days. In particular, if the culturing is performed as a batch process, the culturing time normally ranges from about 12 hours to about 36 hours. When using a continuous process, fermentation times might be up to 21 days or longer.

The purification of the products, L-threonine and L-lysine, from the culture media can be achieved by use of commonly known methods, e.g. by use of ion-exchange resins as described in U.S. Patent No. 5,342,766.

Finally, the present invention relates to the use of an aspartokinase enzyme, which is modified as defined above, a vector comprising a polynucleotide encoding such aspartokinase enzyme and/or a host cell comprising such polynucleotide and/or the vector for producing L-lysine or L-threonine.

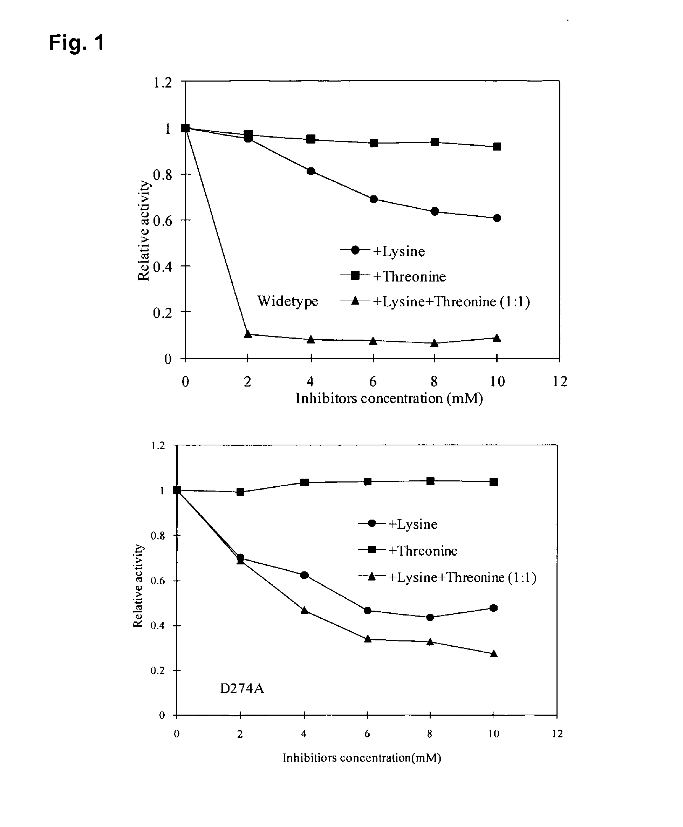

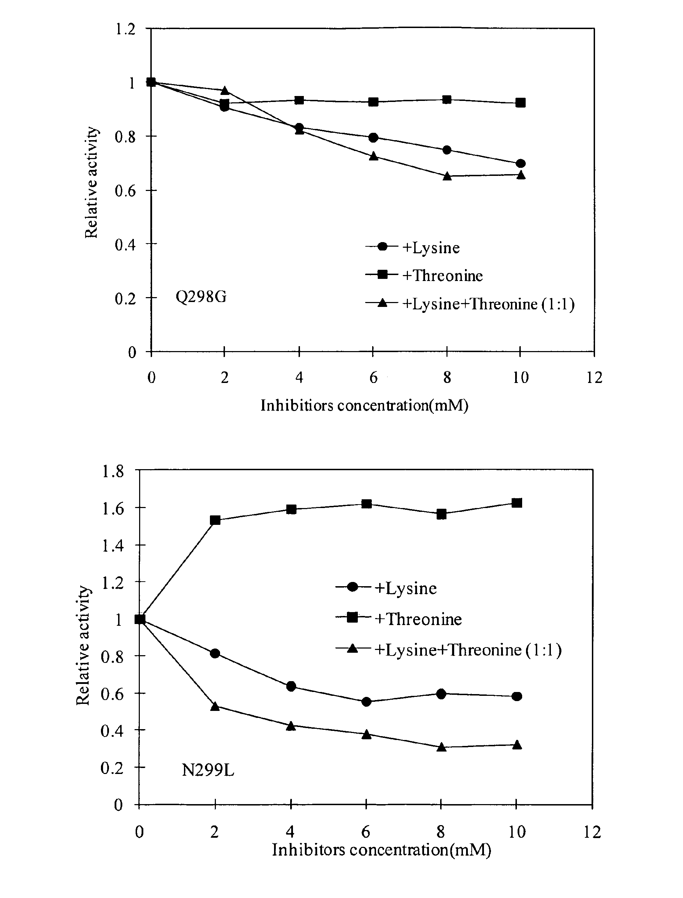

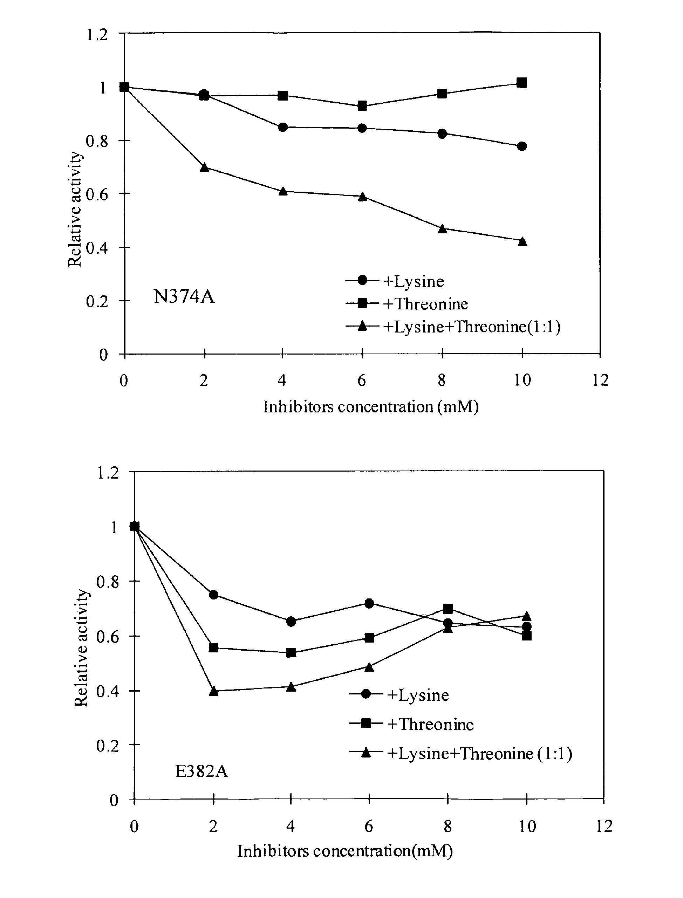

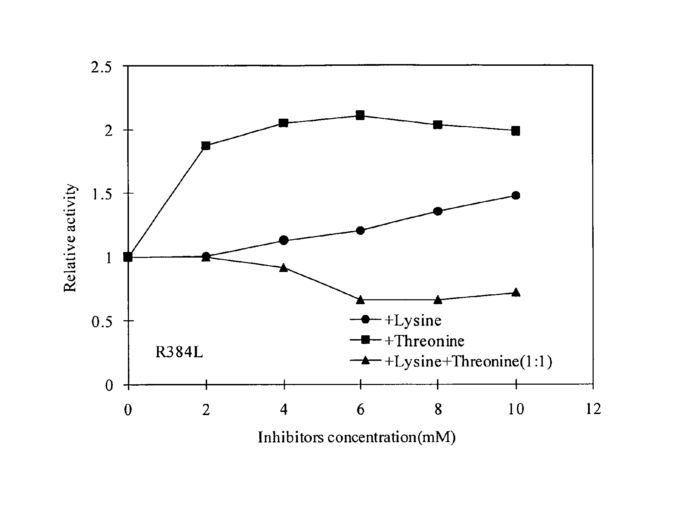

Fig. 1 shows the inhibition profiles of wild-type aspartokinase and aspartokinase mutants of the invention using threonine, lysine and mixtures of L-lysine and L-threonine as inhibitors.

### EXAMPLES

### Example 1: Site-directed mutagenesis of the lysC gene

The lysC gene was amplified from chromosomal DNA of *C. glutamicum* ATCC 1302 by PCR using the following primers:
5'-GAGCCTCATATGGCCCTGGTCGTACAGAAATA-3' (SEQ ID NO:2), and
5'-ATTAATCTCGAGGCGTCCGGTGCCTGCATAAA-3' (SEQ ID NO:3).

The PCR reaction mixture contained:
0.5 µl *C. glutamicum* ATCC 1302 chromosomal DNA (0.1 mg/µl)
0.5 µl primer 1 (SEQ ID NO:2)(10 pm/µl)
0.5 µl primer 2(SEQ ID NO:3)(10 pm/µl)
0.5 µl pfu polymerase (Fermentas Co, LTD, Germany)
5.0 µl 2 mM dNTP
5.0 µl 10 buffer (10x)
38.0 µl Water

The following PCR program was used for amplification:
95°C 5 min
95°C 1 min
60°C 1 min
72°C 2 min (30 cycles)
72°C 15 min
4°C 10 min

The amplified fragment was purified and digested with the restriction enzymes NdeI and XhoI. Subsequently, the digested fragments were ligated with the pET-22b+ vector (Invitrogen GmbH, Germany) that had also been digested by NdeI and *Xho*I. The recombinant plasmid, designated pET-lysC, was transformed into *E. coli* JM109. The recombinant plasmid was purified and it sequenced to confirm the nucleotide sequence.

The plasmid pET-lysC was used as a template for subsequent mutagenesis experiments which introduced site-directed mutations into the amino acid sequence of lysC at a predicted position. The mutagenesis experiments were carried out according to the instruction of QuickChange II XL site-directed mutagenesis kit (Stratagene).

For the mutation of Asp at amino acid position 274 to Ala, the following primers were used:
5'-TTCTGGGTATTTCCGCTAAGCCAGGCGAGGC-3' (SEQ ID NO:4) and
5'-GCCTCGCCTGGCTTAGCGGAAATACCCAGAA-3' (SEQ ID NO:5).

The mutant plasmid resulting from the PCR amplification reaction using the primers of SEQ ID NO:4 and 5 was designated pET-lysC(D274A).

For the mutation of Gln at amino acid position 298 to Gly, the following primers were used:
5'-TCAACATTGACATGGTTCTGGGGAACGTCTCTTCTGTAGAAG-3' (SEQ ID NO:6) and 5'-CTTCTACAGAAGAGACGTTCCCCAGAACCATGTCAATGTTGA-3' (SEQ ID NO:7).

The mutant plasmid resulting from the PCR amplification reaction using the primers of SEQ ID NO:6 and 7 was designated pET-lysC(Q298G).

For the mutation of Asn to Leu at amino acid position 299, a PCR was conducted by using the following oligonucleotide primers:
5'-CATTGACATGGTTCTGCAGCTCGTCTCTTCTGTAGAAGAC-3' (SEQ ID NO:8) and 5'-GTCTTCTACAGAAGAGACGAGCTGCAGAACCATGTCAATG-3' (SEQ ID NO:9)

The plasmid resulting from the PCR amplification reaction using the primers of SEQ ID NO:8 and 9 was pET-lysC(N299L).

For the mutation of Asn to Ala at amino acid position 374, a PCR was conducted by using the following oligonucleotide primers:
5'-TCTGCGCGATGTCAACGTGGCCATCGAATTGATTTCCACC-3' (SEQ ID NO:10)
5'-GGTGGAAATCAATTCGATGGCCACGTTGACATCGCGCAGA-3' (SEQ ID NO:11)

The plasmid resulting from the PCR amplification reaction using the primers of SEQ ID NO:10 and 11 was pET-lysC(N374A).

For the mutation of Glu to Ala at amino acid position 382, a PCR was conducted by using the following oligonucleotide primers:
5'-GAATTGATTTCCACCTCTGCGATTCGTATTTCCGTGCTG-3' and (SEQ ID NO:12) and 5'-CAGCACGGAAATACGAATCGCAGAGGTGGAAATCAATTG-3' (SEQ ID NO:13)

The plasmid resulting from the PCR amplification reaction using the primers of SEQ ID NO:12 and 13 was pET-lysC(E382A).

For the mutation of Arg to Leu at amino acid position 384, a PCR was conducted by using the following oligonucleotide primers:
5'-TTCCACCTCTGAGATTCTTATTTCCGTGCTGATCC-3'and (SEQ ID NO:14) and 5'-GGATCAGCACGGAAATAAGAATCTCAGAGGTGGAA-3' (SEQ ID NO:15)

The plasmid resulting from the PCR amplification reaction using the primers of SEQ ID NO:14 and 15 was pET-lysC(R384L).

The sequence of all plasmids encoding a mutated amino acid sequence of aspartokinase was confirmed by nucleotide sequencing. The plasmids were stored at -80°C or directly used for the subsequent transformation experiments.

### Example 2: Expression of mutated aspartokinase enzymes

The plasmid pET-lysC and the plasmids encoding the mutated aspartokinase genes prepared in accordance with example 1 above pET-lysC(D274A), pET-lysC(Q298G), pET-lysC(N299L), pET-lysC-(N374A), pET-lysC(E382A), and pET-lysC(R384L) were transformed into *E. coli* strain BL21 (DE3) CodonPlus RIPL by heat-shock method according to common protocols. The recombinant *E. coli* strains resulting from the transformation experiments were designated:
*E. coli* lysC(D274A),
*E. coli* lysC(Q298G),
*E. coli* lysC(N299L),
*E. coli* lysC(N374A),
*E. coli* lysC(E382A), and
*E. coli* lysC(R384L),

The recombinant *E. coli* strains listed above were cultured at 37°C in LB medium containing 100 mg/L ampicillin until an OD₆₀₀ of 0.6 was reached. Then, IPTG was added to the medium to a final concentration of 0.1 mM, and the bacteria were cultured at 20°C for another 12-14 h. The *E. coli* cells were then harvested and washed with buffer A (20 mM Tris-HCl, pH 7.5) and suspended in buffer B (20 mM Tris-HCl (pH 7.5), 150 mM NaCl, 0.5 M ammonium sulphate). Suspended cells were disrupted by sonication and centrifuged at 13.000 rpm for 1 h. The supernatant was purified by use of a Ni²⁺-NTA column (GE Healthcare Bio-Sciences, Piscataway, NJ).

### Example 3: Measuring L-Lysine and L-threonine feedback inhibition

The aspartokinase activity was assayed by the method of Black and Wright by estimating the quantity of aspartate hydroxamate formed in the presence of hydroxylamine (S. Black, Methods in Enzymology, Academic Press Inc, New York, Vol. V, p. 820 (1962)). The reaction mixture contained 200 mM Tris-HCl (pH 7.5), 10 mM MgSO₄·6H₂O, 10 mM Asp, 10 mM ATP (adjusted to pH 7.0 with KOH), 160 mM NH₂OH-HCl (neutralized with KOH), appropriate concentrations of lysine and threonine (as inhibitors), and the enzyme. After incubation at 30°C for 30 min, the reaction was stopped by mixing with 5% (w/v) FeCl₃ solution, and the absorbance at 540 nm was monitored.

The enzyme activities of the different aspartokinase mutants with different lysine and threonine concentrations are shown in Fig. 1. The results show that all six mutants (D274A, Q298G, N299L, N374A, E382A, and R384L) have been substantially released from lysine and threonine feedback inhibition even at high lysine and threonine concentration.

### Example 4: Integration of the mutant lysC(Q298G) into the chromosome of Corynebacterium glutamicum

To construct a plasmid-free mutant, the mutant lysC(Q298G) gene was integrated into the chromosome DNA of *C. glutamicum* ATCC 1302 by homologous recombination. To construct a replacement vector for *C. glutamicum,* pET-lysC(Q298A) was used as a template. A PCR was conducted by using the following oligonucleotide primers:
5'-GCGAGGAATTCGTTGGCAGCTGCTTTGAACGCTGAT-3' (SEQ ID NO:16)
5'-GGTATGGATCCCGAACAGTGTCAGCTGGGAAATTGC-3' (SEQ ID NO:17)

The amplified fragment was purified and digested with the restriction enzymes *EcoR*I and *BamH*I. Subsequently, the digested fragment was ligated with the pK18mobsacB vector (Kirchner et al. (2003), J. Biotechnol. 104: 287-299) that had also been digested with *Eco*RI and *BamH*I. The ligation mixture is then transformed into the competent cell of *E*. *coli DH5*α*-mcr* (Grant et al. (1990), Proc. Natl. Acad. Sci. USA. 87:4645-4649). The resulting recombinant plasmid was purified and designated pK18mobsacB-lysC(Q298A). The recombinant plasmid pK18mobsacBlysC(Q298A) was subsequently transformed into wild type *C. glutamicum* ATCC 13032 by electroporation (Van der Rest et al. (1999), Appl. Microbiol. Biotechnol. 52, 541-545). The vector cannot replicate independently in ATCC 13032 and is only retained in the cell after integration into the chromosome as the result of a recombination event. The integrated mutants are selected by plating the mixture on LB agar supplemented with 15 mg/l kanamycin and 50 mg/l nalidixic acid. Kanamycin-resistant mutants are streaked on plates of LB agar supplemented with 25 mg/l of kanamycin and incubated for 24 hours at 33°C.

To select mutants in which excision of the plasmid has taken place as the result of a second recombination event, the clones are cultivated non-selectively in LB liquid medium for 30 hours. Then, the clones are streaked on LB agar supplemented with 10% of sucrose and incubated for 24 hours. Like the starting plasmid pK18mobsacB, plasmid pK18mobsacBlysC(Q298A) contains not only the kanamycin resistance gene but also a copy of the sacB gene coding for the levan sucrase from *Bacillus subtilis.* Expression of the gene, which is inducible by sucrose, leads to the formation of levan sucrase which in turn catalyzes the synthesis of levan, a substance toxic to *C. glutamicum.* Therefore, the only clones that grow on LB agar supplemented with sucrose are those in which the integrated pK18mobsacB-lysC(Q298A) has been excised as a result of a second recombination event. Depending on the location of the second recombination event in relation to the mutation site, allele replacement or incorporation of the mutation takes place in the excision or the original copy remains in the chromosome of the host.
20 colonies were randomly selected and the corresponding lysC genes were amplified and sequenced. Clones with positive integration were designated *C. glutamicum* lysC(Q298A). These clones were stored at -80°C prior to use.

### Example 5: Production of lysine by the mutant Corynebacterium glutamicum lysC(Q298A)

The recombinant *C. glutamicum* strain lysC(Q298G) was cultured to test the production of L-Lysine. The fermentation medium was composed of CSL 5 g/L, MOPO 20 g/L, 50 g/L glucose, 25 g/L (NH₄)₂SO₄, 0.1 g/L KH₂PO₄, 1 g/L MgSO₄·7H₂O, 0.01 g/L FeSO₄·7H₂O, 0.005 g/L MnSO₄·5H₂O, 0.01 g/L CaCl₂·2H₂O, 0.3 mg/L Biotin, 0.3 mg/L thiamine·HCl, 0.4 g/L L-homoserine, 25 g/L CaCO₃ and 100 mg/L ampicillin. The recombinant *C. glutamicum* strain was cultured at 33°C for 48 hours.

The production of L-lysine by the mutant strain and the wild-type strain is summarized in Table 1. It is shown that by introduction of a aspartokinase released from feedback inhition, lysine is accumulated by the mutant strain in the medium. In contrast, the wild-type strain of *C. glutamicum* does not produce any lysine.

**Table 1: Production of lysine in C. glutamicum**

| **Bacterial strain** | **Production L-lysine (g/L)** |
|---|---|
| *C. glutamicum* (wild-type) | 0 |
| *C*. *glutamicum*/lysC (Q298A) | 4.45 |

## Claims

1. Polypeptide which is an enzymatically active aspartokinase with a reduced sensitivity to feedback inhibition by L-lysine or mixtures of L-lysine and L-threonine, said polypeptide comprising the amino acid sequence of SEQ ID NO:1 or an enzymatically active fragment or homolog thereof, wherein one or more amino acids have been replaced at positions that correspond to amino acids 274, 298, 299, 374, 382 and 384 of SEQ ID NO:1.

2. Polypeptide of claim 1, wherein the amino acid replacements are one or more of the following replacements:
(a) Asp at position 274 is modified to Ala, Ile, Leu, Gly, Phe, Trp, Tyr, Val, Pro, Cys, Met, Thr, Glu, Lys, Arg, or His;
(b) Gln at position 298 is modified to Gly, Ala, Leu, Phe, Ile, Trp, Tyr, Val, Arg, Pro, or Cys;
(c) Asn at position 299 is modified to Leu, Ala, Ile, Gly, Trp, Tyr, Ser, His, Val, Pro, or Cys;
(d) Asn at position 374 is modified to Ala, Ile, Leu, Gly, Trp, Tyr, Val, Pro, Cys, Ser, Thr, Met, Gln, Asp, Glu, Lys, Arg, Phe, His;
(e) Glu at position 382 is modified to Ala, Ile, Phe, Gly, Trp, Tyr, Pro, Ser, Leu or Cys;
(f) Arg at position 384 is modified to Leu, Ala, Ile, Gly, Phe, Trp, Tyr, Val, Pro or Cys.

3. Polypeptide of claim 1 or 2, wherein the amino acid replacements are one or more of the following replacements:
(a) Asp 274 to Ala;
(b) Gln 398 to Gly;
(c) Asn 299 to Leu;
(d) Asn 374 to Ala;
(e) Glu 382 to Ala;
(f) Arg 384 to Leu.

4. Polypeptide of claim 1-3, wherein the sensitivity to feedback inhibition by L-lysine or mixtures of L-lysine and L-threonine is reduced at least 2-fold, at least 5-fold, or at least 10-fold.

5. Polypeptide of claim 1-4, wherein said polypeptide is substantially free from feedback inhibition by L-lysine or mixtures of L-lysine and L-threonine.

6. Polynucleotide encoding a polypeptide of claims 1-5.

7. Vector comprising the polynucleotide of claim 6.

8. Host cell comprising the polynucleotide of claim 6 or the vector of claim 7.

9. Host cell of claim 8, wherein the host cell is a prokaryotic cell.

10. Host cell of claim 9, wherein the host cell is a bacterial cell.

11. Host cell of claim 10, wherein the bacterial cell is an *Escherichia* cell.

12. Host cell of claim 10, wherein the bacterial cell is a *Corynebacterium* cell.

13. Method of producing L-lysine and/or L-threonine, comprising the steps of (i) culturing a host cell of any of claims 8-10, which is capable of producing L-lysine and/or L-threonine, in a culture medium under conditions which allow for the expression of a polypeptide of any of claims 1 to 5, and (ii) obtaining L-lysine and/or L-threonine from the culture medium.

14. Use of a polypeptide of any of claims 1 to 5, a vector of claim 7 or a host cell of claims 8-12, for producing L-lysine and/or L-threonine.


SEQUENCE LISTING

<110> Technische Universität Hamburg-Harburg,
TuTech Innovation GmbH

<120> Modified aspartate kinase from Corynebacterium and its application
for amino acid production

<130> P 82235

<160> 17

<170> PatentIn version 3.3

<210> 1
<211> 421
<212> PRT
<213> Corynebacterium glutamicum

<400> 1

Met Ala Leu Val Val Gln Lys Tyr Gly Gly Ser Ser Leu Glu Ser Ala
1               5                   10                  15

Glu Arg Ile Arg Asn Val Ala Glu Arg Ile Val Ala Thr Lys Lys Ala
            20                  25                  30

Gly Asn Asp Val Val Val Val Cys Ser Ala Met Gly Asp Thr Thr Asp
        35                  40                  45

Glu Leu Leu Glu Leu Ala Ala Ala Val Asn Pro Val Pro Pro Ala Arg
    50                  55                  60

Glu Met Asp Met Leu Leu Thr Ala Gly Glu Arg Ile Ser Asn Ala Leu
65                  70                  75                  80

Val Ala Met Ala Ile Glu Ser Leu Gly Ala Glu Ala Gln Ser Phe Thr
                85                  90                  95

Gly Ser Gln Ala Gly Val Leu Thr Thr Glu Arg His Gly Asn Ala Arg
            100                 105                 110

Ile Val Asp Val Thr Pro Gly Arg Val Arg Glu Ala Leu Asp Glu Gly
        115                 120                 125

Lys Ile Cys Ile Val Ala Gly Phe Gln Gly Val Asn Lys Glu Thr Arg
    130                 135                 140

Asp Val Thr Thr Leu Gly Arg Gly Gly Ser Asp Thr Thr Ala Val Ala
145                 150                 155                 160

Leu Ala Ala Ala Leu Asn Ala Asp Val Cys Glu Ile Tyr Ser Asp Val
                165                 170                 175

Asp Gly Val Tyr Thr Ala Asp Pro Arg Ile Val Pro Asn Ala Gln Lys

180                 185                 190

Leu Glu Lys Leu Ser Phe Glu Glu Met Leu Glu Leu Ala Ala Val Gly
        195         .       200                 205

Ser Lys Ile Leu Val Leu Arg Ser Val Glu Tyr Ala Arg Ala Phe Asn
    210                 215                 220

Val Pro Leu Arg Val Arg Ser Ser Tyr Ser Asn Asp Pro Gly Thr Leu
225                 230                 235                 240

Ile Ala Gly Ser Met Glu Asp Ile Pro Val Glu Glu Ala Val Leu Thr
                245                 250                 255

Gly Val Ala Thr Asp Lys Ser Glu Ala Lys Val Thr Val Leu Gly Ile
            260                 265                 270

Ser Asp Lys Pro Gly Glu Ala Ala Lys Val Phe Arg Ala Leu Ala Asp
        275                 280                 285

Ala Glu Ile Asn Ile Asp Met Val Leu Gln Asn Val Ser Ser Val Glu
    290                 295                 300

Asp Gly Thr Thr Asp Ile Thr Phe Thr Cys Pro Arg Ser Asp Gly Arg
305                 310                 315                 320

Arg Ala Met Glu Ile Leu Lys Lys Leu Gln Val Gln Gly Asn Trp Thr
                325                 330                 335

Asn Val Leu Tyr Asp Asp Gln Val Gly Lys Val Ser Leu Val Gly Ala
            340                 345                 350

Gly Met Lys Ser His Pro Gly Val Thr Ala Glu Phe Met Glu Ala Leu
        355                 360                 365

Arg Asp Val Asn Val Asn Ile Glu Leu Ile Ser Thr Ser Glu Ile Arg
    370                 375                 380

Ile Ser Val Leu Ile Arg Glu Asp Asp Leu Asp Ala Ala Ala Arg Ala
385                 390                 395                 400

Leu His Glu Gln Phe Gln Leu Gly Gly Glu Asp Glu Ala Val Val Tyr
                405                 410                 415

Ala Gly Thr Gly Arg
            420


<210>  2
<211>  32
<212>  DNA
<213>  Artificial

<220>
<223>  oligonucleotide primer

<400>  2
gagcctcata tggccctggt cgtacagaaa ta                                    32


<210>  3
<211>  32
<212>  DNA
<213>  Artificial

<220>
<223>  oligonucleotide primer

<400>  3
attaatctcg aggcgtccgg tgcctgcata aa                                    32


<210>  4
<211>  31
<212>  DNA
<213>  Artificial

<220>
<223>  oligonucleotide primer

<400>  4
ttctgggtat ttccgctaag ccaggcgagg c                                     31


<210>  5
<211>  31
<212>  DNA
<213>  Artificial

<220>
<223>  oligonucleotide primer

<400>  5
gcctcgcctg gcttagcgga aatacccaga a                                     31


<210>  6
<211>  42
<212>  DNA
<213>  Artificial

<220>
<223>  oligonucleotide primer

<400>  6
tcaacattga catggttctg ggaacgtctc ttctgtagaa ag                         42


<210>  7
<211>  42
<212>  DNA
<213>  Artificial

<220>
<223>  oligonucleotide primer

<400>  7
cttctacaga agagacgttc cccagaacca tgtcaatgtt ga                          42


<210>  8
<211>  40
<212>  DNA
<213>  Artificial

<220>
<223>  oligonucleotide primer

<400>  8
cattgacatg gttctgcagc tcgtctcttc tgtagaagac                             40


<210>  9
<211>  40
<212>  DNA
<213>  Artificial

<220>
<223>  oligonucleotide primer

<400>  9
gtcttctaca gaagagacga gctgcagaac catgtcaatg                             40


<210>  10
<211>  40
<212>  DNA
<213>  Artificial

<220>
<223>  oligonucleotide primer

<400>  10
tctgcgcgat gtcaacgtgg ccatcgaatt gatttccacc                             40


<210>  11
<211>  40
<212>  DNA
<213>  Artificial

<220>
<223>  oligonucleotide primer

<400>  11
ggtggaaatc aattcgatgg ccacgttgac atcgcgcaga                             40

<210>  12
<211>  39
<212>  DNA
<213>  Artificial

<220>
<223>  oligonucleotide primer

<400>  12
gaattgattt ccacctctgc gattcgtatt tccgtgctg                                39


<210>  13
<211>  39
<212>  DNA
<213>  Artificial

<220>
<223>  oligonucleotide primer

<400>  13
cagcacggaa atacgaatcg cagaggtgga aatcaattg                                39


<210>  14
<211>  35
<212>  DNA
<213>  Artificial

<220>
<223>  oligonucleotide primer

<400>  14
ttccacctct gagattctta tttccgtgct gatcc                                    35


<210>  15
<211>  35
<212>  DNA
<213>  Artificial

<220>
<223>  oligonucleotide primer

<400>  15
ggatcagcac ggaaataaga atctcagagg tggaa                                    35


<210>  16
<211>  36
<212>  DNA
<213>  Artificial

<220>
<223>  oligonucleotide primer

<400>  16
gcgaggaatt cgttggcagc tgctttgaac gctgat                                36

<210>  17
<211>  36
<212>  DNA
<213>  Artificial

<220>
<223>  oligonucleotide primer

<400>  17
ggtatggatc ccgaacagtg tcagctggga aattgc                                36

SEQUENCE LISTING

<110>  Technische Universität Hamburg-Harburg,
       TuTech Innovation GmbH

<120>  Modified aspartate kinase from Corynebacterium and its
       application  for amino acid production

<130>  P 82235

<160>  17

<170>  PatentIn version 3.3

<210>  1
<211>  421
<212>  PRT
<213>  Corynebacterium glutamicum

<400>  1

Met Ala Leu Val Val Gln Lys Tyr Gly Gly Ser Ser Leu Glu Ser Ala
1               5                   10                  15

Glu Arg Ile Arg Asn Val Ala Glu Arg Ile Val Ala Thr Lys Lys Ala
            20                  25                  30

Gly Asn Asp Val Val Val Val Cys Ser Ala Met Gly Asp Thr Thr Asp
        35                  40                  45

Glu Leu Leu Glu Leu Ala Ala Ala Val Asn Pro Val Pro Pro Ala Arg
    50                  55                  60

Glu Met Asp Met Leu Leu Thr Ala Gly Glu Arg Ile Ser Asn Ala Leu
65                  70                  75                  80

Val Ala Met Ala Ile Glu Ser Leu Gly Ala Glu Ala Gln Ser Phe Thr
                85                  90                  95

Gly Ser Gln Ala Gly Val Leu Thr Thr Glu Arg His Gly Asn Ala Arg
            100                 105                 110

Ile Val Asp Val Thr Pro Gly Arg Val Arg Glu Ala Leu Asp Glu Gly
        115                 120                 125

Lys Ile Cys Ile Val Ala Gly Phe Gln Gly Val Asn Lys Glu Thr Arg
    130                 135                 140

Asp Val Thr Thr Leu Gly Arg Gly Gly Ser Asp Thr Thr Ala Val Ala
145                 150                 155                 160

Leu Ala Ala Ala Leu Asn Ala Asp Val Cys Glu Ile Tyr Ser Asp Val
                165                 170                 175

Asp Gly Val Tyr Thr Ala Asp Pro Arg Ile Val Pro Asn Ala Gln Lys
            180                 185                 190

Leu Glu Lys Leu Ser Phe Glu Glu Met Leu Glu Leu Ala Ala Val Gly
        195                 200                 205

Ser Lys Ile Leu Val Leu Arg Ser Val Glu Tyr Ala Arg Ala Phe Asn
    210                 215                 220

Val Pro Leu Arg Val Arg Ser Ser Tyr Ser Asn Asp Pro Gly Thr Leu
225                 230                 235                 240

Ile Ala Gly Ser Met Glu Asp Ile Pro Val Glu Glu Ala Val Leu Thr
                245                 250                 255

Gly Val Ala Thr Asp Lys Ser Glu Ala Lys Val Thr Val Leu Gly Ile
            260                 265                 270

Ser Asp Lys Pro Gly Glu Ala Ala Lys Val Phe Arg Ala Leu Ala Asp
        275                 280                 285

Ala Glu Ile Asn Ile Asp Met Val Leu Gln Asn Val Ser Ser Val Glu
    290                 295                 300

Asp Gly Thr Thr Asp Ile Thr Phe Thr Cys Pro Arg Ser Asp Gly Arg
305                 310                 315                 320

Arg Ala Met Glu Ile Leu Lys Lys Leu Gln Val Gln Gly Asn Trp Thr
                325                 330                 335

Asn Val Leu Tyr Asp Asp Gln Val Gly Lys Val Ser Leu Val Gly Ala
            340                 345                 350

Gly Met Lys Ser His Pro Gly Val Thr Ala Glu Phe Met Glu Ala Leu
        355                 360                 365

Arg Asp Val Asn Val Asn Ile Glu Leu Ile Ser Thr Ser Glu Ile Arg
    370                 375                 380

Ile Ser Val Leu Ile Arg Glu Asp Asp Leu Asp Ala Ala Ala Arg Ala
385                 390                 395                 400

Leu His Glu Gln Phe Gln Leu Gly Gly Glu Asp Glu Ala Val Val Tyr
                405                 410                 415

Ala Gly Thr Gly Arg
            420

<210>  2
<211>  32
<212>  DNA
<213>  Artificial

<220>
<223>  oligonucleotide primer

<400>  2
gagcctcata tggccctggt cgtacagaaa ta                                    32


<210>  3
<211>  32
<212>  DNA
<213>  Artificial

<220>
<223>  oligonucleotide primer

<400>  3
attaatctcg aggcgtccgg tgcctgcata aa                                    32


<210>  4
<211>  31
<212>  DNA
<213>  Artificial

<220>
<223>  oligonucleotide primer

<400>  4
ttctgggtat ttccgctaag ccaggcgagg c                                     31


<210>  5
<211>  31
<212>  DNA
<213>  Artificial

<220>
<223>  oligonucleotide primer

<400>  5
gcctcgcctg gcttagcgga aatacccaga a                                     31


<210>  6
<211>  42
<212>  DNA
<213>  Artificial

<220>
<223>  oligonucleotide primer

<400>  6
tcaacattga catggttctg gggaacgtct cttctgtaga ag                          42


<210>  7
<211>  42
<212>  DNA
<213>  Artificial

<220>
<223>  oligonucleotide primer

<400>  7
cttctacaga agagacgttc cccagaacca tgtcaatgtt ga                         42


<210>  8
<211>  40
<212>  DNA

<213>  Artificial

<220>
<223>  oligonucleotide primer

<400>  8
cattgacatg gttctgcagc tcgtctcttc tgtagaagac                              40


<210>  9
<211>  40
<212>  DNA
<213>  Artificial

<220>
<223>  oligonucleotide primer

<400>  9
gtcttctaca gaagagacga gctgcagaac catgtcaatg                              40


<210>  10
<211>  40
<212>  DNA
<213>  Artificial

<220>
<223>  oligonucleotide primer

<400>  10
tctgcgcgat gtcaacgtgg ccatcgaatt gatttccacc                              40


<210>  11
<211>  40
<212>  DNA
<213>  Artificial

<220>
<223>  oligonucleotide primer

<400>  11
ggtggaaatc aattcgatgg ccacgttgac atcgcgcaga                              40


<210>  12
<211>  39
<212>  DNA
<213>  Artificial

<220>
<223>  oligonucleotide primer

<400>  12
gaattgattt ccacctctgc gattcgtatt tccgtgctg                               39


<210>  13
<211>  39
<212>  DNA
<213>  Artificial

<220>
<223>  oligonucleotide primer

<400>  13
cagcacggaa atacgaatcg cagaggtgga aatcaattg                               39

<210>  14
<211>  35
<212>  DNA
<213>  Artificial

<220>
<223>  oligonucleotide primer

<400>  14
ttccacctct gagattctta tttccgtgct gatcc                                35


<210>  15
<211>  35
<212>  DNA
<213>  Artificial

<220>
<223>  oligonucleotide primer

<400>  15
ggatcagcac ggaaataaga atctcagagg tggaa                                35


<210>  16
<211>  36
<212>  DNA
<213>  Artificial

<220>
<223>  oligonucleotide primer

<400>  16
gcgaggaatt cgttggcagc tgctttgaac gctgat                               36


<210>  17
<211>  36
<212>  DNA
<213>  Artificial

<220>
<223>  oligonucleotide primer

<400>  17
ggtatggatc ccgaacagtg tcagctggga aattgc                               36